Europäisches Patentamt

**European Patent Office** (11) Veröffentlichungsnummer : **0 073 428**

Office européen des brevets **B1**

(19)

(12) # EUROPÄISCHE PATENTSCHRIFT

(45) Veröffentlichungstag der Patentschrift : 12.11.86

(51) Int. Cl.⁴ : **A 61 K 9/16**, A 61 K 9/20, A 61 K 31/71

(21) Anmeldenummer : 82107609.8

(22) Anmeldetag : 20.08.82

(54) **Neue Arzneimittelpräparation für Glykosidhydrolasen-Inhibitoren.**

(30) Priorität : 01.09.81 DE 3134591

(43) Veröffentlichungstag der Anmeldung : 09.03.83 Patentblatt 83/10

(45) Bekanntmachung des Hinweises auf die Patenterteilung : 12.11.86 Patentblatt 86/46

(84) Benannte Vertragsstaaten : AT BE CH DE FR GB IT LI LU NL SE

(56) Entgegenhaltungen :
DE-A- 2 903 710
FR-A- 2 338 707
US-A- 3 995 026
Die Akte enthält technische Angaben, die nach dem Eingang der Anmeldung eingereicht wurden und die nicht in dieser Patentschrift enthalten sind.

(73) Patentinhaber : BAYER AG
Konzernverwaltung RP Patentabteilung
D-5090 Leverkusen 1 Bayerwerk (DE)

(72) Erfinder : Luchtenberg, Helmut, Dr.
Brungsgasse 47
D-5300 Bonn (DE)

EP 0 073 428 B1

Jouve, 18, rue St-Denis, 75001 Paris, France

## Beschreibung

Die vorliegende Erfindung betrifft neue Arzneimittelpräparationen, die als Wirkstoff mindestens einen Glykosidhydrolasen-Inhibitor des Aminozuckertyps und als weitere Zusatzstoffe Stärke, mindestens einen Zusatzstoff aus der Gruppe Magnesiumstearat, Calciumstearat, Stearinsäure und Talkum sowie gegebenenfalls weitere Zusatzstoffe und weniger als 6 Gew.-% Wasser enthalten.

Als Glykosidhydrolasen-Inhibitoren, die im Rahmen der vorliegenden Erfindung Verwendung finden können, kommen insbesondere Akarbose und mit Akarbose verwandte Inhibitoren in Betracht. Solche Inhibitoren sind beschrieben z. B. in der DE-OS 2 347 782, in der DE-OS 2 855 409 sowie in der DE-OS 2 903 710.

Arzneimittelpräparationen, die Glykosidhydrolasen-Inhibitoren vom Aminozuckertyp als Wirkstoff, Stärke und weitere Zusatzstoffe enthalten, sind aus der FR-PS 2 338 707 bekannt.

Die vorliegende Erfindung betrifft Arzneimittelpräparationen, bestehend aus mindestens einem Glykosidhydrolasen-Inhibitor des Aminozuckertyps als Wirkstoff, Stärke und Zusatzstoffen, die dadurch gekennzeichnet sind, daß sie nicht mehr als 6 Gew.-% Wasser und mindestens einen Zusatzstoff aus der Gruppe Magnesiumstearat, Calciumstearat, Stearinsäure und Talkum und gegebenenfalls weitere Zusatzstoffe enthalten, wobei sich die Gewichtsmenge an Wirkstoff zur Gesamtgewichtsmenge der übrigen Zusatzstoffe wie 1 : 1 bis 1 : 25 verhält.

Die erfindungsgemäßen Präparationen eignen sich insbesondere zur Herstellung von Tabletten (Beispiele 1 und 2) sowie zur Herstellung von verkapselten Granulaten (Beispiel 3). Welche weitere Zusatzstoffe gegebenenfalls zugesetzt werden, richtet sich danach, welche zur Endapplikation geeignete Form gewünscht wird.

In den erfindungsgemäßen Arzneimittelpräparationen verhält sich die Gewichtsmenge an Wirkstoff zur Gesamtgewichtsmenge der übrigen Zusatzstoffe wie 1 : 1 bis 1 : 25.

Außerdem enthalten die erfindungsgemäßen Präparationen Stärke. Diese Stärke kann in Form von Kartoffelstärke, Maisstärke, Weizenstärke oder auch Reisstärke zugesetzt werden. Diese Stärke, die vorzugsweise getrocknet ist, wird in einer Gewichtsmenge vom 1- bis 10-fachen des Wirkstoffes zugesetzt.

Das in der Präparation enthaltene Magnesiumstearat, Calciumstearat, Stearinsäure und/oder Talkum wird in einer Gewichtsmenge von 0,1 bis 3 %, bezogen auf den Wirkstoff, vorzugsweise 0,2 bis 1,5 %, insbesondere 1 %, eingesetzt.

Wenn die erfindungsgemäße Präparation nach der Verfahrensweise der Trockengranulation zu einem Granulat verarbeitet wird, so empfiehlt sich der Zusatz von Cellulose. Diese kann in Pulverform zugegeben werden. Besonderes bevorzugt ist mikrokristalline Cellulose. Die Gewichtsmenge der eingesetzten Cellulose beträgt das 0,5- bis 1,5-fache bezogen auf den in der Präparation enthaltenen Wirkstoff.

Außerdem ist es bei Anwendung der Trockengranulation vorteilhaft, ein Silikat, vorzugsweise Siliciumdioxid, einzusetzen. Hier wird kolloidales Siliciumdioxid ganz besonders bevorzugt verwendet. Die Gewichtsmenge an Silikat beträgt 0,5 bis 1,5 % bezogen auf den Wirkstoff, vorzugsweise 1 %.

Bei der Verarbeitung der erfindungsgemäßen Präparation zu einem Granulat nach der Verfahrensweise der Wirbelschichtgranulation ist der Einsatz von Polyvinylpyrrolidon von Vorteil. Dieses PVP hat im allgemeinen ein Molekulargewicht von etwa 15 000 bis etwa 60 000, bevorzugt wird PVP mit einem MG von 20 000 bis 30 000.

Ist die Herstellung eines in Wasser schnell löslichen Granulates angestrebt, so empfiehlt sich, der erfindungsgemäßen Arzneimittelpräparation mindestens einen Zuckeralkohol vom Typ Pentose oder Hexose zuzugeben. Als solcher ist Mannit oder Sorbit bevorzugt. Solche Zuckeralkohole werden im allgemeinen in einer Gewichtsmenge vom 5- bis 15-fachen, vorzugsweise etwa 10-fachen, bezogen auf den Wirkstoff, eingesetzt.

Vorzugsweise verhält sich die Gewichtsmenge an Inhibitor in der erfindungsgemäßen Arzneimittelpräparation zur Gesamtgewichtsmenge der übrigen Zusatzstoffe wie 1 : 2,5 bis 1 : 3,5.

Da die Glykosidhydrolasen-Inhibitoren des Aminozuckertyps hygroskopisch sind und der Feuchtigkeitsgehalt der Präparation einen extremen Einfluß auf die Stabilität, insbesondere die Lagerstabilität der Präparation hat, muß dafür Sorge getragen werden, daß der Feuchtigkeitsgehalt nicht über 6 Gew.-%, vorzugsweise nicht über 5 Gew.-% hinausgeht.

Die absolute Gewichtsmenge der in der erfindungsgemäßen Präparation enthaltenen Wirkstoffe richtet sich nach der gewünschten Therapie. Im allgemeinen beträgt diese absolute Gewichtsmenge 100 mg pro Einzeldosis.

Die nachfolgenden Beispiele geben zugleich ganz besonders bevorzugte erfindungsgemäße Arzneimittelpräparationen wieder.

Beispiel 1

100 kg Akarbose werden mit 108,5 kg getrockneter Stärke, 45 kg mikrokristalliner Cellulose, 0,5 kg kolloidalem Siliciumdioxid und 0,5 kg Magnesiumstearat gemischt und diese Mischung trocken komprimiert. Nach dem Zerbrechen und Sieben der Komprimate wird das so erhaltene Granulat mit 15 kg mikrokristalliner Cellulose und 0,5 kg Magnesiumstearat gemischt und anschließend zu Tabletten von je 0,27 g verpreßt oder in Kapseln abgefüllt.

Beispiel 2

100 kg Akarbose werden mit 94 kg Maisstärke und 40 kg mikrokristalliner Cellulose in einem Wirbeschichtgranulator durch kontinuierliches Sprühen von Wasser und gleichzeitiger Zufuhr von Warmluft granuliert. Die Produkttemperatur sollte hierbei etwa 70 °C nicht unterschreiten. Das so erhaltene Granulat wird gesiebt und nach dem Mischen mit 15 kg mikrokristalliner Cellulose und 1 kg Magnesiumstearat zu Tabletten von 0,25 g verpreßt.

Beispiel 3

100 kg Akarbose werden mit 43,5 kg Maisstärke und 82 kg mikrokristalliner Cellulose in einem Wirbelschichtgranulator durch kontinuierliches Aufsprühen einer wäßrigen Lösung von 8 kg Polyvinylpyrrolidon (PVP-Gehalt : ca. 12 Gew.-%) und gleichzeitiger Zufuhr von Warmluft granuliert. Die Produkttemperatur sollte hierbei etwa 70 °C nicht unterschreiten. Das so erhaltene Granulat wird gesiebt und mit 15 kg getrockneter Maisstärke, 0,5 kg Cellulose und 1 kg Magnesiumstearat gemischt und in Kapseln abgefüllt.

**Patentansprüche** (für die Vertragsstaaten : BE, CH, DE, FR, GB, IT, LI, LU, NL, SE)

1. Arzneimittelpräparation, bestehend aus mindestens einem Glykosidhydrolasen-Inhibitor des Aminozuckertyps als Wirkstoff, Stärke und Zusatzstoffen, dadurch gekennzeichnet, daß die Arzneimittelpräparation nicht mehr als 6 Gew.-% Wasser und mindestens einen Zusatzstoff aus der Gruppe Magnesiumstearat, Calciumstearat, Stearinsäure und Talkum und gegebenenfalls weitere Zusatzstoffe enthält, wobei sich die Gewichtsmenge an Wirkstoff zur Gesamtgewichtsmenge der übrigen Zusatzstoffe wie 1 : 1 bis 1 : 25 verhält.

2. Arzneimittelpräparation nach Anspruch 1, dadurch gekennzeichnet, daß als weiterer Zusatzstoff mindestens ein Stoff aus der Gruppe Silikat, Polyvinylpyrrolidon, Zuckeralkohol und Cellulose enthalten ist.

3. Arzneimittelpräparation nach Anspruch 2, dadurch gekennzeichnet, daß sie Cellulose enthält.

4. Arzneimittelpräparation nach Anspruch 3, dadurch gekennzeichnet, daß sie Cellulose in der 0,5- bis 1,5-fachen Menge des Glykosidhydrolasen-Inhibitors enthält.

5. Arzneimittelpräparation nach Anspruch 3, dadurch gekennzeichnet, daß die Cellulose mikrokristallin ist.

6. Arzneimittelpräparation nach den Ansprüchen 1 bis 2, dadurch gekennzeichnet, daß sie einen Zuckeralkohol vom Typ Pentose oder Hexose enthält.

7. Arzneimittelpräparation nach Anspruch 6, dadurch gekennzeichnet, daß sie den Zuckeralkohol in der 5- bis 15-fachen Menge des Glykosidhydrolasen-Inhibitors enthält.

8. Arzneimittelpräparation nach den Ansprüchen 1 bis 7, dadurch gekennzeichnet, daß sich die Gewichtsmenge an Wirkstoff zur Gesamtgewichtsmenge der übrigen Zusatzstoffe wie 1 : 2,5 bis 1 : 10 verhält.

9. Arzneimittelpräparation nach einem der Ansprüche 1 bis 8 in Tablettenform.

10. Arzneimittelpräparation nach einem der Ansprüche 1 bis 8 in Granulatform.

**Patentansprüche** (für den Vertragsstaat AT)

1. Verfahren zur Herstellung von Arzneimittelpräparationen enthaltend nicht mehr als 6 Gew.-% Wasser, bestehend aus mindestens einem Glykosidhydrolasen-Inhibitor des Aminozuckertyps als Wirkstoff und Stärke, mindestens einem Zusatzstoff aus der Gruppe Magnesiumstearat, Calciumstearat, Stearinsäure und Talkum und gegebenenfalls weiteren Zusatzstoffen, wobei sich die Gewichtsmenge an Wirkstoff zur Gesamtgewichtsmenge der übrigen Zusatzstoffe wie 1 : 1 bis 1 : 25 verhält, dadurch gekennzeichnet, daß man den Wirkstoff mit getrockneter Stärke, mindestens einem Zusatzstoff aus der Gruppe Magnesiumstearat, Calciumstearat, Stearinsäure und Talkum und gegebenenfalls weiteren Zusatzstoffen, wobei sich die Gewichtsmenge an Wirkstoff zur Gesamtgewichtsmenge der übrigen Zusatzstoffe wie 1 : 1 bis 1 : 25 verhält, mischt, die Mischung trocken komprimiert, zerbricht, siebt und gegebenenfalls zu Tabletten verpreßt oder in Kapseln füllt.

2. Verfahren zur Herstellung von Arzneimittelpräparationen enthaltend nicht mehr als 6 Gew.-% Wasser, bestehend aus mindestens einem Glykosidhydrolasen-Inhibitor des Aminozuckertyps als Wirkstoff und Stärke, mindestens einem Zusatzstoff aus der Gruppe Magnesiumstearat, Calciumstearat, Stearinsäure und Talkum und gegebenenfalls weiteren Zusatzstoffen, wobei sich die Gewichtsmenge an Wirkstoff zur Gesamtgewichtsmenge der übrigen Zusatzstoffe wie 1 : 1 bis 1 : 25 verhält, dadurch gekennzeichnet, daß man den Wirkstoff mit Stärke und Cellulose in einem Wirbelschichtgranulator durch kontinuierliches Sprühen von Wasser und gleichzeitige Zufuhr von Warmluft granuliert, das erhaltene Granulat siebt, mit Cellulose und mit mindestens einem Zuschlagstoff aus der Gruppe Magnesiumstearat, Calciumstearat, Stearinsäure und Talkum, gegebenenfalls unter Zusatz weiterer getrockneter Stärke, mischt und gegebenenfalls zu Tabletten verpreßt.

**Claims** (for the Contracting States : BE, CH, DE, FR, GB, IT, LI, LU, NL, SE)

1. A drug preparation consisting of at least one glycoside hydrolase inhibitor of the amino sugar type as the active ingredient, starch and additives, characterised in that the drug preparation contains no more than 6 % by weight of water and at least one additive from the group comprising magnesium stearate, calcium stearate, stearic acid and talcum and optionally further additives, the relationship between the quantity by weight of active ingredient and the total quantity by weight of the other additives being 1 : 1 to 1 : 25.

2. A drug preparation according to Claim 1, characterised in that at least one substance from the group comprising silicate, polyvinylpyrrolidone, sugar alcohol and cellulose is contained therein as the further additive.

3. A drug preparation according to Claim 2, characterised in that it contains cellulose.

4. A drug preparation according to Claim 3, characterised in that it contains cellulose in 0.5 to 1.5 times the quantity of the glycoside hydrolase inhibitor.

5. A drug preparation according to Claim 3, characterised in that the cellulose is microcrystalline.

6. A drug preparation according to Claims 1 to 2, characterised in that it contains a sugar alcohol of the pentose or hexose type.

7. A drug preparation according to Claim 6, characterised in that it contains the sugar alcohol in 5 to 15 times the quantity of the glycoside hydrolase inhibitor.

8. A drug preparation according to Claims 1 to 7, characterised in that the relationship between the quantity by weight of the active ingredient and the total quantity by weight of the other additives is 1 : 2.5 to 1 : 10.

9. A drug preparation according to one of Claims 1 to 8 in tablet form.

10. A drug preparation according to one of Claims 1 to 8 in granular form.

**Claims** (for the Contracting State AT)

1. Process for the production of drug preparations containing no more than 6 % by weight of water, consisting of at least one glycoside hydrolase inhibitor of the amino sugar type as the active ingredient and starch, at least one additive from the group comprising magnesium stearate, calcium stearate, stearic acid and talcum and optionally further additives, the relationship between the quantity by weight of active ingredient and the total quantity by weight of the other additives being 1 : 1 to 1 : 25, characterised in that the active ingredient is mixed with dried starch, at least one additive from the group comprising magnesium stearate, calcium stearate, stearic acid and talcum and optionally further additives, the relationship between the quantity by weight of active ingredient and the total quantity by weight of the other additives being 1 : 1 to 1 : 25, the mixture is dry compressed, broken up, sieved and optionally compressed into tablets or filled into capsules.

2. Process for the production of drug preparations containing no more than 6 % by weight of water, consisting of at least one glycoside hydrolase inhibitor of the amino sugar type as the active ingredient and starch, at least one additive from the group comprising magnesium stearate, calcium stearate, stearic acid and talcum and optionally further additives, the relationship between the quantity by weight of active ingredient and the total quantity by weight of the other additives being 1 : 1 to 1 : 25, characterised in that the active ingredient is granulated with starch and cellulose in a fluidised bed granulotor by continuous spraying of water and simultaneous introduction of hot air, the granules obtained are sieved, mixed with cellulose and with at least one additive from the group comprising magnesium stearate, calcium stearate, stearic acid and talcum, optionally with the addition of further dried starch, and optionally compressed into tablets.

**Revendications** (pour les Etats contractants : BE, CH, DE, FR, GB, IT, LI, LU, NL, SE)

1. Préparation de médicament, constituée par au moins un inhibiteur de glycoside-hydrolase du type aminosucre comme substance active, de l'amidon et des additifs, caractérisée en ce que la préparation de médicament ne contient pas plus de 6 % en poids d'eau et contient au moins un additif du groupe stéarate de magnésium, stéarate de calcium, acide stéarique et talc et, le cas échéant, d'autres additifs, le rapport de la quantité en poids de substance active à la quantité totale en poids des autres additifs ayant une valeur de 1 : 1 à 1 : 25.

2. Préparation de médicament suivant la revendication 1, caractérisée en ce qu'elle contient comme autre additif au moins une substance du groupe silicate, polyvinylpyrrolidone, sucre-alcool et cellulose.

3. Préparation de médicament suivant la revendication 2, caractérisée en ce qu'elle contient de la cellulose.

4. Préparation de médicament suivant la revendication 3, caractérisée en ce qu'elle contient de la cellulose en quantité de 0,5 à 1,5 fois la quantité d'inhibiteur de glycoside-hydrolase.

5. Préparation de médicament suivant la revendication 3, caractérisée en ce que la cellulose est microcristalline.

6. Préparation de médicament suivant les revendications 1 et 2, caractérisée en ce qu'elle contient un sucre-alcool du type pentose ou hexose.

7. Préparation de médicament suivant la revendication 6, caractérisée en ce qu'elle contient le sucre-alcool en quantité de 5 à 15 fois la quantité d'inhibiteur de glycoside-hydrolase.

8. Préparation de médicament suivant les revendications 1 à 7, caractérisée en ce que le rapport de la quantité en poids de substance active à la quantité totale en poids des autres additifs va de 1 : 2,5 à 1 : 10.

9. Préparation de médicament suivant l'une des revendications 1 à 8, sous la forme de comprimés.

10. Préparation de médicament suivant l'une des revendications 1 à 8, sous la forme de granulés.

**Revendications** (pour l'Etat contractant AT)

1. Procédé de production de préparations de médicaments ne contenant pas plus de 6 % en poids d'eau, composées d'au moins un inhibiteur de glycoside-hydrolase du type aminosucre comme substance active et d'amidon, d'au moins un additif du groupe stéarate de magnésium, stéarate de calcium, acide stéarique et talc et, le cas échéant, d'autres additifs, le rapport de la quantité en poids de substance active à la quantité totale en poids des autres additifs ayant une valeur de 1 : 1 à 1 : 25, caractérisé en ce qu'on mélange la substance active avec de l'amidon séché, au moins un additif du groupe stéarate de magnésium, stéarate de calcium, acide stéarique et talc et, le cas échéant, d'autres additifs, le rapport de la quantité en poids de substance active à la quantité totale en poids des autres additifs ayant une valeur de 1 : 1 à 1 : 25, on comprime le mélange à sec, on le fragmente, on le tamise et on le presse éventuellement en comprimés ou on en charge des capsules.

2. Procédé de production de préparations de médicaments ne contenant pas plus de 6 % en poids d'eau, composées d'au moins un inhibiteur de glycoside-hydrolase du type aminosucre comme substance active et d'amidon, d'au moins un additif du groupe stéarate de magnésium, stéarate de calcium, acide stéarique et talc et, le cas échéant, d'autres additifs, le rapport de la quantité en poids de substance active à la quantité totale en poids des autres additifs ayant une valeur de 1 : 1 à 1 : 25, caractérisé en ce qu'on granule la substance active avec de l'amidon et de la cellulose dans un granulateur à couche tourbillonnaire par pulvérisation continue d'eau et arrivée simultanée d'air chaud, on tamise le produit granulé obtenu, on le mélange avec de la cellulose et avec au moins un additif du groupe stéarate de magnésium, stéarate de calcium, acide stéarique et talc et, le cas échéant avec addition d'un supplément d'amidon séché, et on presse éventuellement le mélange en comprimés.